# EUROPÄISCHE PATENTSCHRIFT

(11) **EP 0 725 634 B1**
(45) Veröffentlichungstag und Bekanntmachung des Hinweises auf die Patenterteilung: **16.08.2001**
(21) Anmeldenummer: 94931040.3
(22) Anmeldetag: 04.11.1994
(51) Int. Cl.: A61K 31/13, A61K 31/27, A61K 31/34, A61K 31/41, A61K 31/415, A61K 31/495, A61K 9/70, A61K 47/32

(54) **PFLASTER ZUR BEHANDLUNG VON NAGELMYKOSEN**
PLASTER FOR THE TREATMENT OF NAIL MYCOSES
EMPLATRE UTILISE POUR TRAITER LES MYCOSES DES ONGLES

(30) Priorität: 06.11.1993 DE 4337945
(43) Veröffentlichungstag der Anmeldung: 14.08.1996
(73) Patentinhaber: LABTEC GESELLSCHAFT FüR TECHNOLOGISCHE FORSCHUNG UND ENTWICKLUNG MBH, 40764 Langenfeld (DE)
(72) Erfinder: CORDES, Günter, D-42799 Leichlingen (DE); VOLLMER, Ulrike, D-41236 Mönchengladbach (DE)
(74) Vertreter: König, Beate, Dipl.-Phys. Dr.
(86) Internationale Anmeldenummer: EP9403624
(87) Internationale Veröffentlichungsnummer: WO9512393

(56) Entgegenhaltungen:
- EP-A- 0 241 050
- EP-A- 0 274 863
- DE-A- 4 140 888
- US-A- 4 769 013
- DATABASE WPI Week 8823, Derwent Publications Ltd., London, GB; AN 88-158813 (23) & JP,A,63 099 866 (ISHIDA K.) 2. Mai 1988
- DATABASE WPI Week 8805, Derwent Publications Ltd., London, GB; AN 88-031420 (05) & JP,A,62 289 515 (ISHIDA K.) 16. Dezember 1987
- CHEMICAL ABSTRACTS, vol. 110, no. 10, 6. März 1989, Columbus, Ohio, US; abstract no. 82506, & JP,A,63 109 865 (ORIENTAL PHARMACEUTICAL AND SYNTHETIC CHEMICAL CO. LTD.,JAPAN) 14. Mai 1988
- DATABASE WPI Week 9049, Derwent Publications Ltd., London, GB; AN 90-365884 (49) & JP,A,02 264 723 (TAISHO PHARMACEUT. KK.) 29. Oktober 1990
- DATABASE WPI Week 8306, Derwent Publications Ltd., London, GB; AN 83-13039K (06) & JP,A,57 209 213 (KAKEN CHEM. KK.) 22. Dezember 1982

## Beschreibung

Die Erfindung betrifft ein Pflaster zur Behandlung von Nagelmykosen.

Pilzerkrankungen der Nägel sind eine weit verbreitete Erscheinung. In der medizinischen Fachliteratur findet man Hinweise, daß 8 % der Bevölkerung daran leiden. Hierbei spielt eine Rolle, daß in feuchter Umgebung eine relativ hohe Infektionsgefahr gegeben ist (beispielsweise in Badeanstalten oder beim Tragen von Schuhen oder Schutzhandschuhen). Es handelt sich um eine Erkrankung, die nur schlecht abheilt und einer Behandlung bedarf.

Als Behandlungsmethoden gibt es die Möglichkeit, den befallenen Nagel nach entsprechender Vorbehandlung abzulösen, was aber von vielen Betroffenen wegen der damit verbundenen Unannehmlichkeiten abgelehnt wird. Außerdem sind in den letzten Jahren moderne Antimykotika entwickelt worden, die auch nach oraler Einnahme gegen Nagelpilzerkrankungen wirken. Die Behandlung muß jedoch über eine lange Zeit von einigen Monaten durchgeführt werden, wobei bei der oralen Behandlung Nebenwirkungen auftreten, da der ganze Organismus mit dem Medikament in Kontakt kommt. Die Folge ist, daß der Patient die Therapie abbricht und somit die Pilzerkrankung nicht abheilt.

Es wurden auch Lösungen und sogar Nagellack entwickelt, die pilzhemmende Wirkstoffe enthalten, um sie nur lokal am Ort der Erkrankung freizusetzen, und die damit die Nebenwirkungen der oralen Therapie nicht aufweisen. Allerdings liegt die Pilzbesiedlung im Nagelbett unter der Nagelplatte (vgl. Charlet, Kosmetik für Apotheker, Wissenschaftliche Verlagsgesellschaft mbH, Stuttgart 1989), durch die der Wirkstoff zunächst hindurchgelangen muß. Lösungen werden zu rasch abgespült, beispielsweise beim Waschen oder Duschen, und auch ein Nagellack kann nicht genügend Wirkstoff und Permeationsförderer bzw. Enhancer aufnehmen, also Substanzen, die den Wirkstoff durch den Nagel zum Ort der Pilzerkrankung schleusen. Insofern ist eine wirksame lokale Behandlung bis heute ein Problem.

Erfindungsgemäß wird nun ein Pflaster zur Behandlung von Nagelmykosen vorgeschlagen, das gemäß Patentanspruch 1 definiert ist.

Bevorzugte Ausführungsformen ergeben sich aus den Unteransprüchen.

Es handelt sich dabei um ein Pflaster mit einem Antimykotikum, das auf den befallenen Nagel aufgeklebt wird. Der pilzhemmende Wirkstoff kann dazu in eine Polymermatrix eingebettet werden, die beispielsweise aus einem Acrylat-Mischpolymeren besteht. Ferner können in die Matrix Substanzen eingearbeitet werden, die den Wirkstoff aus der Matrix heraus- und/oder durch den Nagel hindurchbringen können, also sogenannte Permeationsförderer oder Enhancer, die speziell proteinreiche Barrieren der Nägel beeinflussen, beispielsweise das Keratin.

Darüberhinaus können Zusätze zugesetzt werden, beispielsweise Aerosil bzw. für eine Acrylat-Copolymerisation übliche Vernetzer, die die Klebeeigenschaften regulieren. Damit ist das System einschichtig, was den Herstellungsprozess vereinfacht.

Die Matrix kann mit einer Abdeckfolie versehen sein, damit durch die klebenden Eigenschaften der Matrix keine Beeinträchtigung beim Tragen erfolgt und die Matrix mit den in ihr enthaltenen Substanzen beim Waschen nicht abgetragen wird.

Ein weiterer Vorteil des erfindungsgemäßen Pflasters ist in der Möglichkeit zu sehen, ausreichend viel Wirkstoff in die Matrix einzubetten, um den Fluß des Wirkstoffs durch den Nagel lange aufrechtzuerhalten, was eine zur Pilzbekämpfung wichtige Eigenschaft darstellt.

Ein erfindungsgemäßes Pflaster kann 1,1 bis 1,7 mg Wirkstoff pro cm² enthalten, so daß auf den Nagel je nach individueller Größe in der Regel eine Dosis von 3 bis 6 mg appliziert wird. Das erfindungsgemäße Pflaster kann dabei so konzipiert werden, daß das Pflaster mehrere Tage auf dem Nagel verbleiben kann, was der Patienten-Compliance entgegenkommt und die Behandlungskosten senkt.

Bei der Herstellung eines erfindungsgemäßen Pflasters wird besonderes Augenmerk auf die Auswahl der Polymere , der Permeationsförderer, der Klebeeigenschaft und der Abdeckfolie gelegt. Die Polymere sollen eine ausreichende Menge des Wirkstoffs in gelöster Form aufnehmen und die Permeationsförderer müssen die Permeation des Wirkstoffs durch den Nagel verbessern. Die wirkstoffhaltige Klebermatrix weist erfindungsgemäß eine starke Klebkraft auf dem Nagel auf, ist nach dem Tragen jedoch abziehbar. Erfindungsgemäß wird eine kosmetisch einwandfreie Abdeckfolie vorgesehen, die eine genügende Flexibilität aufweist, um sich dem Nagel gut anzupassen, auch wenn der Nagel eine unregelmäßige Oberfläche besitzt. Die Abdeckfolie besteht vorzugsweise aus Weich-PVC, das insbesondere hautfarben pigmentiert ist. Auf diese Folie kann gegebenenfalls handelsüblicher kosmetischer Nagellack aufgetragen werden, ohne daß dadurch die Therapie der Nagelmykose beeinflußt wird.

Nachstehend wird die Erfindung durch ein Beispiel näher erläutert.

### Beispiel 1

Zur Herstellung von 1.800 cm² Pflasterfläche werden 2,5 g Miconazol soweit möglich in Ethylacetat unter Rühren gelöst. Man führt 0,9 g Dimethylsulfoxid zu, rührt erneut, gibt sodann 60 g einer 49,1-proz. Lösung von Durotak 901-1052 und rührt bis zu Homogenität. Danach wird auf eine beidseitig silikonisierte Polyesterfolie (75 *µ*m Dicke) ausgestrichen, so daß die Naßfilmdicke 500 *µ*m beträgt. Nach dem Trocknen über 2 h im Trockenschrank bei 60 °C und Lagerung bei 25 °C über 12 h wird mit einem Polyolefin-Film einer Dicke von 50 *µ*m (Cotran 9722) kaschiert. Anschließend werden mit einer Stanze 4,5 cm² große Pflaster ausgestanzt. Beim Applizieren schneidet der Patient das Pflaster auf die jeweilige Größe des Nagels zu.

## Patentansprüche

1. Pflaster zum Aufkleben auf den menschlichen Nagel, anfweisend eine flexible Abdeckfolie, eine einschichtige Polymermatrix, die mit der Abdeckfolie verbunden ist und eine antimykotisch wirksame Substanz (ausgenommen Clotrimazol und Bifonazol) (Wirkstoff) und eine die Permeation des Wirkstoffes durch den Nagel fördernde Substanz (Permeationsförderer) enthält, sowie eine Schutzschicht, wobei die Polymermatrix keines Zusatzes von Weichmachern bedarf und erhältlich ist durch radikalische Polymerisation von 50 bis 80 % 2-Ethylhexylacrylat, 5 bis 20 % Butylacrylat, 20 bis 40 % Methylacrylat, 2 bis 8 % Acrylsäure, 2 bis 30 % Vinylacetat, 0,5 bis 3 % Hydroxyethylacrylat und 0,02 bis 0,1 % Glycidylmethacrylat oder Mischungen dieser substanzen sowie gegebenenfalls anderen Substanzen, insbesondere Aerosil und Vernetzern in einer Menge von bis zu 5 % (auf Gewichtsbasis und bezogen auf die Polymermatrix ohne Wirkstoff und Permeationsförderer) und die flexible Abdeckfolie aus Weich-PVC oder Polyolefin besteht.

2. Pflaster gemäß Anspruch 1, **gekennzeichnet** durch eine Polymermatrix auf Acrylatbasis für ein Pflaster von 3 bis 6 cm² Fläche, insbesondere von Nagelgröße, erhältlich durch Polymerisation von 70 bis 80 % 2-Ethylhexylacrylat, 12 bis 18 % Butylacrylat, 2,8 bis 6,8 % Acrylsäure und 3 bis 7 % Vinylacetat.

3. Pflaster gemäß Anspruch 1, **gekennzeichnet** durch eine Polymermatrix auf Acrylatbasis für ein Pflaster von 3 bis 6 cm² Fläche insbesondere von Nagelgröße, erhältlich durch Polymerisation von 72 bis 83 % 2-Ethylhexylacrylat, 1,5 bis 3,3 % Acrylsäure, 15 bis 25 % Vinylacetat und gegebenenfalls 0,1 bis 0,8 % Aluminium-acetylacetonat und/oder 0,1 bis 0,35 % Titan-acetylacetonat.

4. Pflaster nach einem der Ansprüche 1 bis 3, dadurch gekennzeichnet, daß als Permeationsförderer verwendet werden können Sulfoxide, insbesondere DMSO und Decylmethylsulfoxid; Säureamide wie Pyrrolidone, Dimethylformamid und Dimethylacetamid; Alkylsulfonate, insbesondere Natriumdodecylsulfat; N-Acetyl-L-cystein; Polyalkohole, insbesondere Propylenglycol; Keratolytica, insbesondere Salicylsäure; Harnstoff; und/oder Milchsäure.

5. Pflaster nach einem der Ansprüche 1 bis 3, dadurch gekennzeichnet, daß es sich bei dem Wirkstoff handelt um Miconazol, Econazol, Isoconazol, Tioconazol, Terconazol, Oxiconazol, Ketoconazol und/oder Itraconazol.

6. Pflaster nach einem der Ansprüche 1 bis 4, dadurch gekennzeichnet, daß es sich bei dem Wirkstoff handelt um Tolciclat, Sulbentin, Haloprogin, Griseofulvin, Cyclopirox, gegebenenfalls in Form ihrer Salze, und/oder Terbinafin.

7. Pflaster nach einem der Ansprüche 1 bis 6, dadurch **gekennzeichnet**, daß als Permeationsförderer verwendet werden Dimethylsulfoxid in einer Konzentration in der Matrix von 1 bis 6 % oder Milchsäure in einer Konzentration von 1 bis 6 % (jeweils Konzentration in der Matrix).

## Claims

1. Plaster for sticking onto the human nail, characterized by a flexible covering film, a single-layer polymer matrix which is linked with the covering film, and contains an antimycotically active substance (excluding clotrimazole and bifonazole) (active compound) and a substance promoting the permeation of the active compound through the nail (permeation promoter), as well as a protective layer, whereby the polymer matrix needs no addition of plasticizers and is obtainable by free-radical polymerization of 50 to 80 % of 2-ethylhexyl acrylate, 5 to 20 % of butyl acrylate, 20 to 40 % of methyl acrylate, 2 to 8 % of acrylic acid, 2 to 30 % of vinyl acetate, 0.5 to 3 % of hydroxyethyl acrylate and 0.02 to 0.1 % of glycidyl methacrylate or mixtures thereof, and, optionally, other substances, in particular Aerosil and crosslinkers, in an amount of up to 5 % (on a weight basis and relative to the polymer matrix without active compound and permeation promoter) and whereby the flexible cover film consists of soft PVC or polyolefin.

2. Plaster according to Claim 1, characterized by a polymer matrix based on acrylate for a plaster of 3 to 6 cm² surface area, in particular having the size of a nail, obtainable by polymerization of 70 to 80 % of 2-ethylhexyl acrylate, 12 to 18 % of butyl acrylate, 2.8 to 6.8 % of acrylic acid and 3 to 7 % of vinyl acetate.

3. Plaster according to Claim 1, characterized by a polymer matrix based on acrylate for a plaster of 3 to 6 cm² surface area, in particular having the size of a nail, obtainable by polymerization
of 72 to 83 % of 2-ethylhexyl acrylate, 1.5 to 3.3 % of acrylic acid, 15 to 25 %
of vinyl acetate and optionally 0.1 to 0.8 % of aluminium acetylacetonate and/or 0.1 to 0.35 % of titanium acetylacetonate.

4. Plaster according to one of Claims 1 to 3, characterized in that the permeation promoters used can be sulphoxides, in particular DMSO and decylmethyl sulphoxide; acid amides, such as pyrrolidones, dimethylformamide and dimethylacetamide; alkylsulphonates, in particular sodium dodecyl sulphate; N-acetyl-L-cysteine; polyalcohols, in particular propylene glycol; keratolytics, in particular salicylic acid; urea; and/or lactic acid.

5. Plaster according to one of Claims 1 to 3, characterized in that the active compound is miconazole, econazole, isoconazole, tioconazole, terconazole, oxiconazole, ketoconazole and/or itraconazole.

6. Plaster according to one of Claims 1 to 4, characterized in that the active compound is tolciclate, sulbentine, haloprogin, griseofulvin, cyclopirox, optionally in the form of their salts, and/or terbinafine.

7. Plaster according to one of Claims 1 to 6, characterized in that the permeation promoters used are dimethyl sulphoxide in a concentration of I to 6 % or lactic acid in a concentration of 1 to 6 % (in each case concentration in the matrix).

## Revendications

1. Pansement à coller sur l'ongle humain, comprenant une feuille de recouvrement souple, une matrice polymère monocouche, qui est liée à la feuille de recouvrement et qui contient une substance à action anti-mycosique (le Clotrimazol et le Bifonazol étant exclus) (substances actives) et une substance (agent favorisant l'infiltration) favorisant l'infiltration de la substance active à travers l'ongle, ainsi qu'une couche protectrice, la matrice polymère ne requérant aucune addition d'assouplisseurs et étant obtenue au moyen d'une polymérisation par voie radicalaire de 50 à 80 % d'acrylate de 2-éthylhexyle, 5 à 20 % d'acrylate de butyle, 20 à 40 % d'acrylate de méthyle, 2 à 8 % d'acide acrylique, 2 à 30 % d'acétate de vinyle, 0,5 à 3 % d'acrylate d'hydroxyéthyle et 0,02 à 0,1 % de méthacrylate de glycidyle ou des mélanges de ces substances ainsi qu'éventuellement d'autres substances, en particulier l'Aérosil et des agents de réticulation en une quantité allant jusqu'à 5 % (sur la base du poids et rapportés à la matrice polymère sans substance active et sans agent favorisant l'infiltration) et la feuille de recouvrement souple étant constituée de PVC mou ou de polyoléfine.

2. Pansement selon la revendication 1, caractérisé par une matrice polymère à base d'acrylate pour un pansement d'une surface de 3 à 6 cm², en particulier de la taille d'un ongle, que l'on peut obtenir par polymérisation de 70 à 80 % d'acrylate de 2-éthylhexyle, 12 à 18 % d'acrylate de butyle, 2,8 à 6,8 % d'acide acrylique, et 3 à 7 % d'acétate de vinyle.

3. Pansement selon la revendication 1, caractérisé par une matrice polymère à base d'acrylate pour un pansement d'une surface de 3 à 6 cm², en particulier de la taille d'un ongle, que l'on peut obtenir par polymérisation de 72 à 83 % d'acrylate de 2-éthylhexyle, 1,5 à 3,3 % d'acide acrylique, 15 à 25 % d'acétate de vinyle et éventuellement 0,1 à 0,8 % d'acétylacétonate d'aluminium et/ou 0,1 à 0,35 % d'acétylacétonate de titane.

4. Pansement selon l'une quelconque des revendications 1 à 3, caractérisé en ce que des sulfoxydes, en particulier le DMSO et le sulfoxyde de décylméthyle ; des amides d'acide comme les pyrrolidones, le diméthylformamide et le diméthylacétamide ; des sulfonates d'alkyle, en particulier le docécylsulfate de sodium ; la N-acétyl-L-cystéine ; des polyols, en particulier le propylèneglycol ; des kératolytiques, en particulier l'acide salicylique ; l'urée ; et/ou l'acide lactique peuvent être employés comme agents favorisant l'infiltration.

5. Pansement selon l'une quelconque des revendications 1 à 3, caractérisé en ce que, quant à la substance active, il s'agit de Miconazole, Econazole, Isoconazole, Tioconazole, Terconazole, Oxiconazole, Kétoconazole et/ou Itraconazole.

6. Pansement selon l'une quelconque des revendications 1 à 4, caractérisé en ce que, quant à la substance active, il s'agit de Tolciclate, Sulbentine, Haloprogine, Griséofulvine, Cyclopyrox, éventuellement sous forme de leurs sels, et/ou de Terbinafine.

7. Pansement selon l'une quelconque des revendications 1 à 6, caractérisé en ce que le sulfoxyde de diméthyle a une concentration dans la matrice de 1 à 6 % ou l'acide lactique dans une concentration de 1 à 6 % (respectivement concentration dans la matrice) sont employés comme agents favorisant l'infiltration.
